# EUROPEAN PATENT SPECIFICATION

(11) **EP 1 667 745 B1**
(45) Date of publication and mention of the grant of the patent: **16.12.2009**
(21) Application number: 04782578.1
(22) Date of filing: 30.08.2004
(51) Int. Cl.: A61L 27/34, A61L 29/08, A61L 31/10

(54) **LUBRICIOUS COATING**
GLEITÜBERZUG
REVETEMENT LUBRIFIANT

(30) Priority: 09.09.2003 US 658718
(43) Date of publication of application: 14.06.2006
(73) Proprietor: Boston Scientific Limited, St. Michael, Barbados, West Indies (BB)
(72) Inventor: KANGAS, Steve, Woodbury, MN 55125 (US)
(74) Representative: Altenburg, Udo
(86) International application number: PCT/US2004/028134
(87) International publication number: WO 2005/025631

(56) References cited:
- EP-A- 0 483 941
- EP-A- 0 496 305
- EP-A- 0 586 324
- EP-A- 0 747 071
- WO-A-03/093357
- WO-A-2004/014448
- WO-A-2004/014449
- WO-A-2004/014451
- US-A- 4 100 309
- US-A- 4 408 026
- US-A- 4 642 267
- US-A- 5 985 955
- US-B1- 6 238 799

## Description

Improving the lubricity of insertable medical devices such as by application of lubricious polymeric coatings to the surfaces of such devices for the purpose of reducing friction when the device is introduced into the human body, generally referred to as lubricious coatings, is known in the art.

Catheters and other medical devices used for introduction in blood vessels, urethra, body conduits and the like and guide wires used with such devices are examples of article which may be provided with hydrophilic coatings. Guide catheters, and catheters for balloon angioplasty and biopsy are specific examples of such catheters.

Silicone has been used as a coating for many olefin and metallic medical devices. However, silicone is hydrophobic, and although imparting some lubricity against certain surfaces, silicone's coefficient of friction increases dramatically in the presence of water, plasma, or blood.

Hydrogel polymers have also been used in coatings. Depending on their composition hydrogels are characterized by an initial non-tacky to tacky quality followed by lubricity upon hydration.

Hydrogel polymers used in coatings for medical devices are known in the prior art. For example the US 6,238,799, EP0 483 941, US 4,642,267, US 4,100,309 and EP 0 747 071 disclose different kinds of lubricious coatings for medical devices.

Starting from the prior art the problem to be solved by this invention is to find a medical device comprising a lubricious coating which coefficient of friction is advantageous and which properties can be modified even when applied to the surface of the medical device. This problem is solved by claim 1 of the invention.

In one aspect, the present invention relates to a medical device having a lubricious coating, the lubricious coating including at least one ethylenically unsaturated resin and at least one hydrophilic polyurethane.

The lubricious coating may be used on guide wires, catheter shafts, and dilatation balloons.

Suitably, the polyurethane is an aliphatic polyether polyurethane.

In some embodiments, the ethylenically unsaturated resin includes at least one mono-, di- or tri- (meth)acrylate.

In one embodiment, a blend of neopentyl glycol diacrylate or polyethylene glycol diacrylate are employed in combination with at least one hydrophilic aliphatic polyether polyurethane. The hydrophilic aliphatic polyether polyurethane may be employed in combination with a second polyurethane polymer which absorbs less water by weight that the hydrophilic aliphatic polyether polyurethane.

The lubricious coatings according to the present invention find utility for reducing frictional forces of insertable medical devices where one surface is movably in contact with another surface.

### BRIEF DESCRIPTION OF THE DRAWINGS

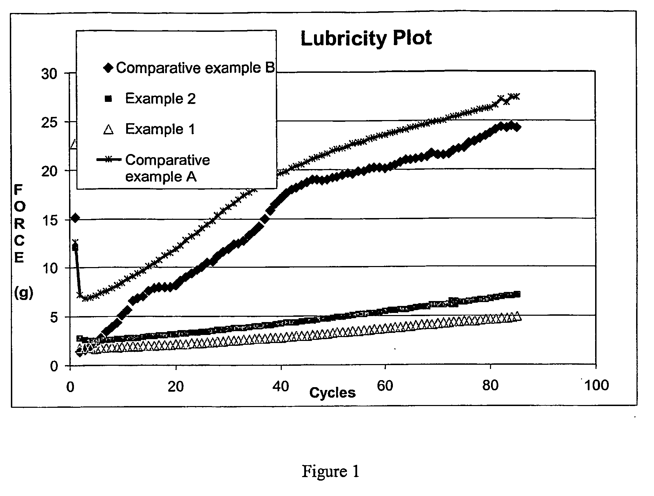

Fig. 1 is a graph showing lubricity and durability of compositions according to the invention as well as comparative examples.

### DETAILED DESCRIPTIONS OF THE PREFERRED EMBODIMENTS

While this invention may be embodied in many different forms, there are described in detail herein specific embodiments of the invention. This description is an exemplification of the principles of the invention and is not intended to limit the invention to the particular embodiments illustrated.

The hydrophilic polyurethanes suitable for use herein are those having a high degree of water absorbancy being capable of absorbing as much as about 500% to about 2000% of their own weight in water.

Suitably, the polyurethane is a thermoplastic polyurethane.

Thermoplastic polyether polyurethanes are a suitable class of polyurethanes, and in particular, aliphatic polyether polyurethanes are suitable for use herein. Examples of such thermoplastic polyurethanes include, but are not limited to, TECOGEL® 500 and TECOGEL® 2000 available from Thermedics, Inc.

Suitable polymers are water swellable, but not water soluble.

Hydrophilic polyurethanes are typically formed with relatively higher amounts of polyethylene oxide or polyethylene glycol.

The highly water absorbent polyurethanes described above, can also be employed in combination with other, less hydrophilic polyurethanes. Examples of suitable polyurethanes are Tecophilic® hydrophilic polyurethanes available from Thermedics, Inc.

Of course, any lubricious polymer may be employed in combination with the hydrophilic polyurethanes described herein. The list of available polymeric materials is vast and such polymeric materials are known to those of ordinary skill in the art.

As used herein, the term ethylenically unsaturated resin, shall be used to refer to any material which has the property of undergoing a chemical reaction which is initiated upon exposure to heat, catalyst, actinic radiation, moisture, etc., to become a relatively insoluble material which, once set, cured or cross-linked, will decompose rather than melt. Typically, such materials referred to herein, may develop a well-bonded three-dimensional structure upon curing.

Any ethylenically unsaturated resin suitable for forming an interpenetrating network (IPN) or semi-interpenetrating network with the hydrophilic polyurethane may be employed herein. Suitably, the crosslinker does not react with the polyurethane.

Suitable radical cure resins include those which are polyfunctional, ethylenically unsaturated compounds such as those under the category of vinyl resins. Examples of suitable resins include, for example, the acrylic esters or acrylates. Examples of such acrylic esters include the (meth)acrylates including mono-, di-, and tri(meth)acrylates and polyacrylates. Examples of suitable members of this class include, but are not limited to, butyl (meth)acrylate, methyl (meth)acrylate, ethyl (meth)acrylate, propyl (meth)acrylate, octyl (meth)acrylate, heptyl (meth)acrylate, nonyl (meth)acrylate, hexyl (meth)acrylate, n-hexyl (meth)acrylate, isopropyl (meth)acrylate, isobutyl (meth)acrylate, decyl (meth)acrylate, isodecyl (meth)acrylate, lauryl (meth)acrylate, stearyl (meth)acrylate, behenyl (meth)acrylate and melissyl (meth)acrylate, methoxyethyl (meth)acrylate, hydroxyl ethyl (meth)acrylate, glycidyl (meth)acrylate, 2-ethylhexyl (meth)acrylate, 2-ethoxyethyl (meth)acrylate, ethylene glycol di(met4)acrylate, propylene glycol di(meth)acrylate, diethylene glycol di(meth)acrylate, polyethylene glycol di(meth)acrylate, 1,5-pentanediol di(meth)acrylate, neopentyl glycol di(meth)acrylate (NPG), 1,6-hexanediol (meth)acrylate, 1,6-hexandiol di(meth)acrylate, polyethylene glycol di(meth)acrylate, polypropylene glycol di(meth)acrylate, pentaerythritol tri(meth)acrylate, trimethylolpropane tri(meth)acrylate, trimethylolpropane dipentaerythritol penta(meth)acrylate, pentaerythritol tetra(meth)acrylate, triethylene glycol di(meth)acrylate, n-butyl (meth)acrylate, benzoin (meth)acrylate, glyceryl propoxy tri(meth)acrylate, 1,3-propylene glycol di(meth)acrylate, tripropylene glycol di(meth)acrylate, 1,3-butylene glycol di(meth)acrylate, 1,4-butanediol di(meth)acrylate, 1,6-hexanediol di(meth)acrylate, diethylene glycol di(meth)acrylate, triethylene glycol di(meth)acrylate, tetraethylene glycol di(meth)acrylate, methyl ethacrylate, ethoxylated bisphenol-A-di(meth)acrylate, and so forth. This list is intended for illustrative purposes only, and is not intended to limit the scope of the present invention. One of ordinary skill in the art would know such materials.

Acrylic nitriles also find utility herein. Examples are the α,β-olefinically unsaturated nitriles including the monoolefinically unsaturated nitriles having from 3 to 10 carbon atoms such as acrylonitrile, methacrylonitrile, and the like.

Illustrative amides include acrylamide, methacrylamide, N-t-butyl acrylamide, N-cyclohexyl acrylamide, methylene-bis-acrylamide, trimethylene-bis-acrylamide, hexamethylene-bis-acrylamide, N,N-dimethylacrylamide and N,N-diethylacrylamide, m-phenylene-bis-acrylamide, p-phenylene-bis-acrylamide, N-methylol-acrylamide, diacetone-acrylamide, butoxymethyl acrylamide, and so forth.

N-alkylol amides of α,β-olefinically unsaturated carboxylic acids including those having from 4 to 10 carbon atoms such as N-methylol acrylamide, N-propanol acrylamide, N-methylol methacrylamide, N-methylol maleimide, N-methylol maleamic acid esters, N-methylol-p-vinyl benzamide, and the like find utility herein.

(Meth)acrylic acids find utility herein.

Other examples include, but are not limited to, N-acrylamido-morpholine, N-acrylamido-piperidine, acrylic acid anilide, methacrylic acid anilide, divinyl benzene, styrene, methyl styrene, butadiene, isoprene, vinyl functional silicones, chlorostyrene, methoxystyrene, chloromethylstyrene, vinyl toluene, 1-vinyl-2-methylimidazole, 1-vinyl-2-undecylimidazole, 1-vinyl-2-undecylimidazoline, N-vinylpyrrolidone, N-vinylcarbazole, vinylbenzyl ether, bis(4-acryloxypolyethoxyphenyl)propane, vinyl ethers, vinylphenyl ether, vinyl esters, carboxylic acids, N,N'-diacrylamidopiperazine, pentaerythritol tetra-allyl ether, and so forth, to mention only a few.

Suitable resins are described in EP 0 363 460 B1, US 4051195, US 2895950, US 3218305, US 3425988, US 5693034, US 6558798, US 6583214, for example, each of which is incorporated by reference herein in its entirety.

Any suitable copolymers of the above-described compounds with other monomers containing polymerizable vinyl groups also find utility herein.

The amount and types of resins that may be employed are too vast to list. Thus, the above lists are intended for illustrative purposes only, and are not intended to limit the scope of the present invention. Other suitable materials would also find utility herein. Such materials are known to those of ordinary skill in the art.

Other examples include, but are not limited to, thermoset resins such as epoxies, unsaturated polyesters, and isocyante based prepolymers.

The above-described ethylenically unsaturated resins may include both one-part and two-part systems, although the one-part systems are desirably employed herein.

In preparing the solution mixture of the present invention, the hydrophilic polyurethane may be mixed with the ethylenically unsaturated resin in a solvent or cosolvent mixture. Examples of suitable organic solvents of a more polar nature include, but are not limited to, the lower alcohols including, but not limited to, isopropyl alcohol and methanol; water; linear or cyclic carboxamides such ad N,N-dimethylacetamide (DMAC), N,N-diethylacetamide, dimethylformamide (DMF), diethylformamide or 1-methyl-2-pyrrolidone (NMP); dimethylsulphoxide (DMSO); and so forth.

Other suitable organic solvents include, but are not limited to, aliphatic, cycloaliphatic or aromatic ether-oxides, more particularly dipropyl oxide, diisopropyl oxide, dibutyl oxide, methyltertiobutylether, ethylene glycol dimethylether (glyme), diethylene glycol dimethylether (diglyme); phenyl oxide; dioxane, tetrahydrofuran (THF). Of course, mixtures of solvents may also be employed.

The above lists are intended for illustrative purposes only and are not intended to limit the scope of the present invention. Other solvents not listed herein would find utility in the invention as well and are known to those of skill in the art.

Crosslinking for UV curable compositions may be facilitated by the addition of a small amount of a photoinitiator such as a free radical initiator or cationic photoinitiators as are commonly used for UV curing. Examples of suitable photoinitiators include, but are not limited to, aromatic-aliphatic ketone derivatives, including benzoin and its derivatives, 2-phenyl-1-indanone, and so forth.

Specific examples of a useful photoinitiator include, but are not limited to, 2,2' dimethoxy-2-phenylacetophenone (IRGACURE® 651), 1-benzoyl-2-hydroxy propane (DAROCUR® 1173), a morpholinoketone (IRGACURE® 369), a bisacylphosphine oxide (IRGACURE® 819), all available from Ciba® Specialty Chemicals, and 2,4,6 dimethylbenzoyl(diphenyl)phosphine oxide (LUCIRIN® TPO available from BASF).

The mixture may then be applied to a substrate out of solvent. The lubricious coating may then be coated onto a surface out of solvent using any coating method known in the art such as dipping, spraying, painting, sponge coating, and so forth.

Crosslinkers which have a higher molecular weight and which are not highly volatile, can be compounded directly with a thermoplastic polyurethane, allowing for coextrusion of the coating.

The solvent may then be allowed to dry. The coating may be dried at room temperature. However, improved durability may be achieved by drying the coating at elevated temperatures of, for example, 70°C. Suitably, drying is conducted at an elevated temperature over several hours to improve the durability of the coating. Once a coating has been applied to a substrate, the coating may then be crosslinked by exposing the coating to heat or actinic radiation such as UV light for a short period of time. This can then trigger the polymerization and crosslinking of the ethylenically unsaturated resin or prepolymer. Suitably the mixture is cured using a high intensity ultraviolet lamp.

The crosslinked structure helps to retain the hydrophilic polyurethane on surfaces to which the coating is applied.

The lubricious coatings according to the invention find utility in the medical device industry, in particular for medical devices inserted in the body. The lubricious coatings find utility on catheter devices, in particular, on guide wires, catheter shafts, and dilatation balloons,

Dilatation balloons may be coated on the body, cone and/or waist portions or any combination thereof. In some embodiments, the balloon is coated on the distal and proximal waist cones, and on a portion of the body, but not in the center of the body. This has been found to reduce "*watermelon seeding*", a term of art used to refer to slippage of the balloon during inflation in a lesion. This can be an issue in particular when the lesion is tapered, but this is not the only situation where "watermelon seeding" can occur.

The lubricity of the coating may be controlled by adding different polyurethanes or other polymers to the blend. This can allow for the use of different coatings on different portions of a catheter device where higher or lower lubricity may be desirable. For example, it may be desirable to coat the proximal portion of the catheter device with a less lubricious formula for better gripping, and to coat the distal portion of the device with a more highly lubricious coating for better trackability. This may be advantageous for guide wires or PV catheter assemblies.

In one embodiment, the distal portion is coated with a ethylenically unsaturated resin and a highly water absorbent aliphatic polyether polyurethane and the proximal portion is coated with a ethylenically unsaturated resin and a blend of a highly water absorbent thermoplastic aliphatic polyether polyurethane and a less water absorbent polymer such as a less water absorbent polyurethane.

The coating according to the present specification may be employed for drug delivery. A drug can be incorporated into the polymer network formed by the crosslinked material which helps to entrap a drug(s) which can then more slowly leach out of the crosslinked network when the medical device is employed in the body.

The following non-limiting examples further illustrate the present invention.

### EXAMPLES

### Example 1

TECOGEL® 2000 polyether polyurethane available from Thermedics, Inc. and neopentylglycol diacrylate (NPGDA (700 MW)) (90/10) was added to a cosolvent blend of isopropyl alcohol (IPA) and water to prepare a 5% solution of TECOGEL® 2000 and NPGDA in 3.75 IPA:1 water. IRGACURE® 369 photoinitiator was added at a 2% loading.

### Example 2

TECOGEL® 2000 polyether polyurethane and polyethyleneglycol diacrylate (PEGDA) (90/10) was added to a cosolvent blend of isopropyl alcohol (IPA) and water to prepare a 5% solids mixture of TECOGEL® 2000 and PEGDA in 3.75 IPA:1 water. IRGACURE® 369 photoinitiator was added at a 2% loading.

### Comparative Example A

A mixture of, polyethylene oxide having a molecular weight of about 90,000 g/mole and NPGDA (10:1) in a cosolvent blend of 3.75:1 isopropyl alcohol (IPA) to water was used to form a 2% solids mixture in solvent. The mixture was applied to a balloon formed of PEBAX® 7033 as described above. Azobisisibutironitrile photoinitiator (2%) was also added in a minimal amount effective to initiate NPG polymerization. This composition is a standard in the industry.

A 2% solids mixture was employed for comparative A versus examples 1 and 2 due to the fact that a 5% solids mixture of examples 1 and 2 is comparable in coating thickness to a 2% solids mixture of comparative A. The molecular weight of TECOGEL® 2000 requires a higher solids content to attain the same coating thickness because it has a lower viscosity than the polyethylene oxide employed in comparative example A.

Each of the above coating compositions were sponge coated on helium plasma treated catheter shafts formed from Pebax 7233 and allowed to dry for several minutes at room temperature. The coatings were cured for 30 sec using a Hg vapor arc lamp.

### Comparative Example B

A 5% solids solution of TECOGEL® 2000 was prepare in a cosolvent blend of 3.75:1 IPA to water. No crosslinker was employed. This solution was applied to a dilatation balloon formed form PEBAX® 7033 polyether block amide copolymer. The coating was allowed to dry at room temperature for 1 hour and 45minutes.

Lubricity was measured using a device that cycles a latex pad along the length of a catheter. The catheter was immersed in water. The latex pad was affixed to an armature which was further connected to a force gauge. An 80 g weight was placed on the armature. The catheter was then cycled back and forth across the pad by a motor drive. Force was measured as a function of the number of cycles. The lower the force, the greater the lubricity. The results are shown in Fig. 1.

The lubricity of comparative examples A and B was initially good, but exhibited poor durability.

Addition of NPGDA or PEGDA to the TECOGEL® 2000 polyurethane showed significant improved in both initial lubricity as well as in durability, i.e. final lubricity which was 5-6 grams. This is due to enhancement to the durability of the polyurethane by entanglement of the themoplastic polyurethane with the cross-linked acrylate network (semi-IPN).

The above disclosure is intended to be illustrative and not exhaustive. The description will suggest many variations and alternatives to those of ordinary skill in the art. All of these alternatives and variations are intended to be included within the scope of the attached claims. Those familiar with the art may recognize other equivalents to the specific embodiments described herein which equivalents are also intended to be encompassed by the claims attached hereto.

## Claims

1. A medical device comprising a lubricious coating, said lubricious coating comprising at least one ethylenically unsaturated resin and at least one aliphatic polyether polyurethane wherein said at least one aliphatic polyether polyurethane does not crosslink, wherein said at least one ethylenically unsaturated resin comprises functional groups which are photochemically activatable, said medical device further comprising at least one photoinitiator, wherein said at least one aliphatic polyether polyurethane is capable of absorbing about 100% to about 2000% of its own weight in water, wherein said medical device is a catheter assembly, wherein said lubricious coating is on a guide wire, dilatation balloon, catheter shaft or combination thereof, and wherein the ethylenically unsaturated resin develops a well-bonded three-dimensional structure upon curing, and wherein the ethylenically unsaturated resin is forming an interpenetrating network or semi-interpenetrating network with the hydrophilic polyurethane employed herein.

2. The medical device of claim 1 wherein said at least one ethylenically unsaturated resin comprises functional groups which are activatable by ultraviolet radiation.

3. The medical device of claim 1 wherein said at least one aliphatic polyether polyurethane is capable of absorbing about 200% to about 2000% of its own weight in water.

4. The medical device of claim 1 wherein said at least one aliphatic polyether polyurethane is capable of absorbing about 500% to about 2000% of its own weight in water.

5. The medical device of claim 1 wherein said at least one ethylenically unsaturated resin comprises at least one member selected from the group consisting of mono-, di- and tri- acrylates, polyacrylates and mixtures thereof.

6. The medical device of claim 1 wherein said at least one ethylenically unsaturated resin is a diacrylate.

7. The medical device of claim 6 wherein said at least one ethylenically unsaturated resin comprises at least one member selected from the group consisting of butyl (meth)acrylate, methyl (meth)acrylate, ethyl (meth)acrylate, propyl (meth)acrylate, octyl (meth)acrylate, heptyl (meth)acrylate, nonyl (meth)acrylate, hexyl (meth)acrylate, n-hexyl (meth)acrylate, isopropyl (meth)acrylate, isobutyl (meth)acrylate, decyl (meth)acrylate, isodecyl (meth)acrylate, lauryl (meth)acrylate, stearyl (meth)acrylate, behenyl (meth)acrylate and mellissyl (meth)acrylate, methoxyethyl (meth)acrylate, hydroxyl ethyl (meth)acrylate, glycidyl (meth)acrylate, 2-ethylhexyl (meth)acrylate, 2-ethoxyethyl (meth)acrylate, ethylene glycol di(meth)acrylate, propylene glycol di(meth)acrylate, diethylene glycol di(meth)acrylate, polyethylene glycol di(meth)acrylate, 1,5-pentanediol di(meth)acrylate, neopentyl glycol di(meth)acrylate (NPG), 1,6-hexanediol (meth)acrylate, 1,6-hexandiol di(meth)acrylate, polyethylene glycol di(meth)acrylate, polypropylene glycol di(meth)acrylate, pentaerythritol tri(meth)acrylate, trimethylolpropane tri(meth)acrylate, trimethylolpropane dipentaerythritol penta(meth)acrylate, pentaerythritol tetra(meth)acrylate, triethylene glycol di(meth)acrylate, n-butyl (meth)acrylate, benzoin (meth)acrylate, glyceryl propoxy tri(meth)acrylate, 1,3-propylene glycol di(meth)acrylate, tripropylene glycol di(meth)acrylate, 1,3-butylene glycol di(meth)acrylate, 1,4-butanediol di(meth)acrylate, 1,6-hexanediol di(meth)acrylate, diethylene glycol di(meth)acrylate, triethylene glycol di(meth)acrylate, tetraethylene glycol di(meth)acrylate, methyl ethacrylate, ethoxylated bisphenol-A-di(meth)acrylate, and mixtures thereof.

8. The medical device of claim 7 wherein said at least one crosslinkable material is selected from the group consisting of neopentyl glycol diacrylate, polyethylene glycol diacrylate and mixtures thereof.

9. The medical device of claim 1 wherein said at least one ethylenically unsaturated resin comprises at least one member selected from the group consisting of styrene, divinyl benzene, acrylamides, carboxylic acid, (meth)acrylic acids, and mixtures thereof.

## Patentansprüche

1. Medizinische Vorrichtung, umfassend einen Gleitüberzug, wobei der Gleitüberzug mindestens ein ethylenisch ungesättigtes Harz und mindestens ein aliphatisches Polyetherpolyurethan umfasst, wobei das mindestens eine aliphatische Polyetherpolyurethan nicht vernetzt, wobei das mindestens eine ethylenisch ungesättigte Harz funktionelle Gruppen umfasst, die photochemisch aktivierbar sind, wobei die medizinische Vorrichtung des Weiteren mindestens einen Photoinitiator umfasst, wobei das mindestens eine aliphatische Polyetherpolyurethan in der Lage ist, etwa 100% bis etwa 2000% seines Eigengewichts in Wasser zu absorbieren, wobei es sich bei der medizinischen Vorrichtung um eine Katheteranordnung handelt, wobei sich der Gleitüberzug auf einem Führungsdraht, einem Dilatations-Ballon, einem Katheterschaft oder einer Kombination davon befindet und wobei das ethylenisch ungesättigte Harz beim Aushärten eine gut verbundene dreidimensionale Struktur entwickelt und wobei das ethylenisch ungesättigte Harz mit dem hier eingesetzten hydrophilen Polyurethan ein durchdringungendes Netzwerk oder ein halbdurchdringendes Netzwerk bildet.

2. Medizinische Vorrichtung nach Anspruch 1, wobei das mindestens eine ethylenisch ungesättigte harz funktionelle Gruppen umfasst, die durch Ultraviolettstrahlung aktivierbar sind.

3. Medizinische Vorrichtung nach Anspruch 1, wobei das mindestens eine aliphatische Polyetherpolyurethan in der Lage ist, etwa 200% bis etwa 2000% seines Eigengewichts in Wasser zu absorbieren.

4. Medizinische Vorrichtung nach Anspruch 1, wobei das mindestens eine aliphatische Polyetherpolyurethan in der Lage ist, etwa 500% bis etwa 2000% seines Eigengewichts in Wasser zu absorbieren.

5. Medizinische Vorrichtung nach Anspruch 1, wobei das mindestens eine ethylenisch ungesättigte Harz mindestens einen Vertreter, ausgewählt aus der Gruppe, bestehend aus Mono-, Di- und Triacrylaten, Polyacrylaten und Gemischen davon, umfasst.

6. Medizinische Vorrichtung nach Anspruch 1, wobei es sich bei dem mindestens einen ethylenisch ungesättigten Harz um ein Diacrylat handelt.

7. Medizinische Vorrichtung nach Anspruch 6, wobei das mindestens eine ethylenisch ungesättigte Harz mindestens einen Vertreter, ausgewählt aus der Gruppe, bestehend aus Butyl(meth)acrylat, Methyl(meth)acrylat, Ethyl(meth)acrylat, Propyl(meth)acrylat, Octyl(meth)acrylat, Heptyl(meth)acrylat, Nonyl(meth)acrylat, Hexyl(meth)acrylat, n-Hexyl(meth)acrylat, Isopropyl(meth)acrylat, Isobutyl(meth)acrylat, Decyl(meth)acrylat, Isodecyl(meth)acrylat, Lauryl(meth)acrylat, Stearyl(meth)acrylat, Behenyl(meth)acrylat und Mellissyl(meth)acrylat, Methoxyethyl(meth)acrylat, Hydroxyethyl(meth)acrylat, Glycidyl(meth)acrylat, 2-Ethylhexyl(meth)acrylat, 2-Ethoxyethyl(meth)acrylat, Ethylenglycoldi(meth)acrylat, Propylenglycoldi(meth)acrylat, Diethylenglycoldi(meth)acrylat, Polyethylenglycoldi(meth)acrylat, 1,5-Pentandioldi(meth)acrylat, Neopentylglycoldi(meth)acrylat (NPG), 1,6-Hexandiol(meth)acrylat, 1,6-Hexandioldi(meth)acrylat, Polyethylenglycoldi(meth)acrylat, Polypropylenglycoldi(meth)acrylat, Pentaerythritoltri(meth)acrylat, Trimethylolpropantri(meth)acrylat, Trimethylolpropandipentaerythritolpenta(meth)acrylat, Pentaerythritoltetra(meth)acrylat, Triethylenglycoldi(meth)acrylat, n-Butyl(meth)acrylat, Benzoin(meth)acrylat, Glycerylpropoxytri(meth)acrylat, 1,3-Propylenglycoldi(meth)acrylat, Tripropylenglycoldi(meth)acrylat, 1,3-Butylenglycoldi(meth)acrylat, 1,4-Butandioldi(meth)acrylat, 1,6-Hexandioldi(meth)acrylat, Diethylenglycoldi(meth)acrylat, Triethylenglycoldi(meth)acrylat, Tetraethylenglycoldi(meth)acrylat, Methylethacrylat, ethoxyliertem Bisphenol-A-di(meth)acrylat und Gemischen davon, umfasst.

8. Medizinische Vorrichtung nach Anspruch 7, wobei das mindestens eine vernetzbare Material ausgewählt ist aus der Gruppe, bestehend aus Neopentylglycoldiacrylat, Polyethylenglycoldiacrylat und Gemischen davon.

9. Medizinische Vorrichtung nach Anspruch 1, wobei das mindestens eine ethylenisch ungesättigte Harz mindestens einen Vertreter, ausgewählt aus der Gruppe, bestehend aus Styrol, Divinylbenzol, Acrylamiden, Carbonsäure, (Meth)acrylsäuren und Gemischen davon, umfasst.

## Revendications

1. Dispositif médical comprenant un revêtement lubrifiant, ledit revêtement lubrifiant comprenant au moins une résine à insaturation éthylénique et au moins un polyéther-polyuréthanne aliphatique, ledit au moins un polyéther-polyuréthanne aliphatique ne subissant pas de réticulation, l'au moins une résine à insaturation éthylénique comprenant des groupes fonctionnels activables par voie photochimique, ledit dispositif médical comprenant en outre au moins un photoa-morceur, ledit au moins un polyéther-polyuréthanne aliphatique étant capable d'absorber d'environ 100 % à environ 2000 % de son poids propre en eau, ledit dispositif médical étant un ensemble de type cathéter, ledit revêtement lubrifiant se trouvant sur un fil guide, un ballonnet de dilatation, une gaine de cathéter ou une combinaison de ceux-ci, la résine à insaturation éthylénique développant une structure tridimensionnelle bien liée après durcissement, la résine à insaturation éthylénique formant un réseau interpénétrant ou un réseau semi-interpénétrant avec le polyuréthanne hydrophile utilisé dans l'invention.

2. Dispositif médical selon la revendication 1, dans lequel l'au moins une résine à insaturation éthylénique comprend des groupes fonctionnels qui sont activables par un rayonnement ultraviolet.

3. Dispositif médical selon la revendication 1, dans lequel ledit au moins un polyéther-polyuréthanne aliphatique peut absorber d'environ 200 % à environ 2000 % de son poids propre en eau.

4. Dispositif médical selon la revendication 1, dans lequel l'au moins un polyéther-polyuréthanne aliphatique est capable d'absorber d'environ 500 à environ 2000 % de son poids propre en eau.

5. Dispositif médical selon la revendication 1, dans lequel ladite au moins une résine à insaturation éthylénique comprend au moins un membre choisi dans le groupe consistant en les mono-, les di- et les triacrylates, les polyacrylates et leurs mélanges.

6. Dispositif médical selon la revendication 1, dans lequel ladite au moins une résine à insaturation éthylénique est un diacrylate.

7. Dispositif médical selon la revendication 6, dans lequel ladite au moins une résine à insaturation éthylénique comprend au moins un membre choisi dans le groupe consistant en le (méth)acrylate de butyle, le (méth)acrylate de méthyle, le (méth)acrylate d'éthyle, le (méth)acrylate de propyle, le (méth)acrylate d'octyle, le (méth)acrylate d'heptyle, le (méth)acrylate de nonyle, le (méth)acrylate d'hexyle, le (méth)acrylate de n-hexyle, le (méth)acrylate d'isopropyle, le (méth)acrylate d'isobutyle, le (méth)acrylate de décyle, le (méth)acrylate d'isodécyle, le (méth)acrylate de lauryle, le (méth)acrylate de stéaryle, le (méth)acrylate de béhényle, et le (méth)acrylate de mellissyle, le (méth)acrylate de méthoxyéthyle, le (méth)acrylate d'hydroxyéthyle, le (méth)acrylate de glycidyle, le (méth)acrylate de 2-éthylhexyle, le (méth)acrylate de 2-éthoxyéthyle, le di(méth)acrylate d'éthylèneglycol, le di(méth)acrylate de propylèneglycol, le di(méth)acrylate de diéthylèneglycol, le di(méth)acrylate de polyéthylène-glycol, le di(méth)acrylate de 1,5-pentanediol, le di(méth)acrylate de néopentylglycol (NPG), le (méth)acrylate de 1,6-hexanediol, le di(méth)acrylate de 1,6-hexanediol, le di(méth)acrylate de polyéthylèneglycol, le di(méth)acrylate de polypropylèneglycol, le tri(méth)acrylate de pentaérythritol, le tri(méth)acrylate de triméthylolpropane, le penta(méth)acrylate de triméthylolpropane-dipentaérythritol, le tétra(méth)acrylate de pentaérythritrol, le di(méth)acrylate de triéthylèneglycol, le (méth)acrylate de n-butyle, le (méth)acrylate de benzoïne, le propoxy-tri(méth)acrylate de glycéryle, le di(méth)acrylate de 1,3-propylèneglycol, le di(méth)acrylate de tripropylène-glycol, le di(méth)acrylate de 1,3-butylèneglycol, le di(méth)acrylate de 1,4-butanediol, le di(méth)acrylate de 1,6-hexanediol, le di(méth)acrylate de diéthylène-glycol, le di(méth)acrylate de triéthylèneglycol, le di(méth)acrylate de tétraéthylèneglycol, l'éthacrylate de méthyle, le di(méth)acrylate de bisphénol-A éthoxylé et les mélanges de ceux-ci.

8. Dispositif médical selon la revendication 7, dans lequel ledit au moins un matériau réticulable est choisi dans le groupe consistant en le diacrylate de néopentyl-glycol, le diacrylate de polyéthylèneglycol et les mélanges de ceux-ci.

9. Dispositif médical selon la revendication 1, dans lequel ladite au moins une résine à insaturation éthylénique comprend au moins un membre choisi dans le groupe consistant en le styrène, le divinylbenzène, les acrylamides, l'acide carboxylique, les acides (méth)acryliques et les mélanges de ceux-ci.
